(19) Europäisches Patentamt
European Patent Office
Office européen des brevets

(11) Veröffentlichungsnummer: **0 191 285**
**B1**

(12) **EUROPÄISCHE PATENTSCHRIFT**

(45) Veröffentlichungstag der Patentschrift: **22.11.90**

(51) Int. Cl.$^5$: **A 61 K 7/06,** C 07 C 69/92

(21) Anmeldenummer: **86100132.9**

(22) Anmeldetag: **07.01.86**

# (54) Sebosuppressive kosmetische Mittel, enthaltend Benzoesäureesterderivate, sowie neue Benzoesäureesterderivate.

(30) Priorität: **14.01.85 DE 3500971**

(43) Veröffentlichungstag der Anmeldung:
**20.08.86 Patentblatt 86/34**

(45) Bekanntmachung des Hinweises auf die Patenterteilung:
**22.11.90 Patentblatt 90/47**

(84) Benannte Vertragsstaaten:
**AT BE CH DE FR GB IT LI NL SE**

(56) Entgegenhaltungen:
**EP-A-0 054 174**
**EP-A-0 114 051**
**WO-A-82/04189**
**US-A-2 154 598**
**US-A-3 716 644**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Möller, Hinrich, Dr.**
**Schumannstrasse 11**
**D-4019 Monheim (DE)**
Erfinder: **Wallat, Siegfried, Dr.**
**Marie-Curie-Strasse 9**
**D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 191 285 B1

**Beschreibung**

Die Erfindung betrifft topische, kosmetische Mittel zur Verbesserung des fettigen und unästhetischen Aussehens der Haare und der Haut, welche bestimmte Benzoesäureesterderivate enthalten. Die Erfindung betrifft ferner neue Benzosäureesterderivate.

Die moderne Kosmetik ist bemüht, das durch übermäßig starke Absonderung der Talgdrüsen und der Kopfhaut verursachte fettige und wenig ästhetisch Aussehen der Haare zu vermindern. Es ist daher vielfach versucht worden, durch geeignete Mittel die Sekretion der Talgdrüsen zu normalisieren, um den Haar wieder sein normales Aussehen zu geben. So wurden zur Bekämpfung der Seborrhoe des beharrten Kopfes kosmetische Pflegemittel mit Schwefel-, Quecksilber- oder Teerzusatz verwendet. Dabei hat sich gezeigt, daß diese Zusätze bei längerer Anwendung häufig zu Nebenwirkungen führen, ohne daß wirklich befriedigende Ergebnisse im Hinblick auf Wirksamkeit und anwendungstechnische·Eigenschaften erzielt werden konnten. Mehrfach sind auch bereits Derivate von Benzosäureestern als antiseborrhoische Zusätze für kosmetische Mittel vorgeschlagen worden (DE—A—31 21 064, DE—A 33 01 313, =EP—A—114051), doch besteht weiterhin der Wunsch nachy Mitteln mit erhöhter sebosuppressiver Wirksamkeit.

Aufgabe der Erfindung ist es, ein kosmetisches Mittel bereitzustellen, welches gegenüber den bekannten Präparaten eine verstärke Wirkung, ohne nachteilige Folgen auf den menschlichen Körper hat.

Es wurde nun gefunden, daß bestimmte Benzoesäureesterderivate hervorragende antiseborrhoische Effekte auch bei sehr geringer Dosierung aufweisen. Gegenüber den bekannten Derivaten von Benzoesäureestern ist die Wirkung überraschenderweise erheblich verbessert, was einen geringeren Mengeneinsatz in den betreffenden sebosuppressiven kosmetischen Mitteln ermöglicht.

Gegenstand der Erfindung sind sebosuppressive kosmetische Mittel, die gekennzeichnet sind durch einen Gehalt an Benzoesäureesterderivaten der allgemeinen Formel

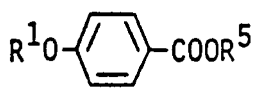

worin bedeuten

(I) $R^1$ = verzweigtkettiges $C_6$—$C_{18}$-Alkyl, $C_3$—$C_9$-Alkylbenzyl, oder

(II) $R^1$ = $R^2$—(O—Y)$_n$—

$R^2$ = $C_6$—$C_{18}$-Alkyl, $C_3$—$C_9$-Alkylbenzyl

Y = $CHR^3$—$CHR^4$,

$R^3$, $R^4$ = H, Methyl

n = 1 oder 2

$R^5$ = $C_1$—$C_4$-Alkyl, Methoxy-, Ethoxy alkyl, $C_1$—$C_4$-Hydroxyalkyl

Die anspruchsgemäß zu verwendenden Verbindungen sind neu. Sie können nach bekannten Verfahren hergestellt werden, z.B. durch Alkylierung bzw. Aralkylierung der entsprechenden 4-Hydroxybenzoesäureester mit Halogeniden oder Etherhalogeniden, die Reste entsprechend $R^1$ enthalten, oder durch Veresterung von in 4-Stellung durch Reste entsprechend $R^1$ substituierten Benzoesäuren mit Hydroxyalkylverbindungen entsprechend $R^5$. Spezielle Ester können auch durch Umesterung erhalten werden.

Beispielsweise können die durch Veresterung hergestellten Verbindungen folgende Säurekomponenten enthalten:

(I) für Reste $R^1$ = verzweigtkettiges $C_6$—$C_{18}$-Alkyl oder $C_3$—$C_9$-Alkylbenzyl:

4-(3,3-Dimethyl-1-butoxy)-, 4-(3,3-Dimethyl-2-butoxy)-, 4-(2-Methyl-1-, -2- bzw. -3-pentoxy)-, 4-(3- bzw. -4-Methyl-1-pentoxy)-4-(3-Ethyl-3-pentoxy)-, 4-(2- bzw. -3-Hexoxy)-, 4-(2-Heptoxy)-, 4-(4-Heptoxy)-, 4-(2,4,4-Trimethyl-1-pentoxy)-, 4-(2-, -3- bzw. -4-Octyloxy)-, 4-(2-Ethyl-hexyloxy)-, 4-(3,5,5-Trimethyl-1-hexyloxy-, 4-(3- bzw. -5-Nonyloxy)-, 4-(3,7-Dimethyl-1-octyloxy)-, 4-(5-Undecyloxy)-, 4-(2,4,6-Trimethyl-1-nonyloxy)-, 4-(2-Butyl-1-octyloxy), 4-Isotridecyloxy-, 4-(2-Hexyl-1-decyloxy)-, 4-Isooctadecyloxy-, 4-[2-(4,4-Dimethyl-2-pentyl)-5,7,7-trimethyl-1-octyloxy]-, 4-(3- bzw. -4-Isopropyl-benzyloxy)-, 4-(2-, -3- bzw. -4-tert. Butylbenzyloxy)-, 4-[4-(3,3-Dimethyl-1-butyl)-benzyloxy]-, 4-[4-(2,4,4-Trimethyl-1-pentyl)-benzyloxy]-, 4-[4-(2-Ethylhexyl)-benzyloxy]-, 4-[4-(3,5,5-Trimethyl-hexyl)-benzyloxy]-benzoesäure.

Bevorzugt werden Verbindungen, für die $R^1$ = verzweigtkettiges $C_8$—$C_{13}$-Alkyl oder $C_3$—$C_6$-Alkylbenzyl, insbesondere mehrfach verzweigtes $C_8$—$C_{13}$-Alkyl ist.

(II) für Reste $R^1$ = $R^2$ —(O—Y)$_n$:

4-(2-Octyloxy-ethoxy)-, 4-(1-Nonyloxy-2-propoxy)-, 4-(2-Decyloxy-1-propoxy)-, 4-(2-Undecyloxy-ethoxy)-, 4-(2-Dodecyloxy-ethoxy)- 4-[2-(2-Dodecyloxy-ethoxy)ethoxy]-, 4-(1-Dodecyloxy-2-propoxy)-, 4-[1-(1-Tetradecyloxy-2-propoxy)-2-propoxy]-, 4-(2-Hexadecyloxyethoxy)-, 4-[2-(2-Hexadecyloxy-ethoxy)-ethoxy]-benzoesäure.

Bevorzugt werden Verbindungen, für die $R^2$ = $C_9$—$C_{14}$-Alkyl und n = 1 ist.

Beispiele für geeignete Reste $R^5$ sind:

Methyl, Ethyl, Propyl, Isopropyl, Butyl, 2-Butyl, 2-Hydroxy-ethyl oder -propyl, 2-Methoxy-ethyl oder -propyl, 2-Ethoxy-ethyl, 3-Hydroxy-propyl oder -butyl.

Die Verbindungen besitzen eine ausgeprägte sebosuppressive Wirkung bei ausgezeichneter Haut-, und

Schleimhautverträglichkeit. Sie lassen sich ohne Schwierigkeit in verschiedene kosmetische Zubereitungen, wie wäßrige oder alkoholische Lösungen, Öle, Suspensionen, Gele, Emulsionen, Salben oder Aerosole einarbeiten, wobei ihre größere Wasserlöslichkeit und Hydrolysestabilität gegenüber den entsprechenden Estern von besonderem Vorteil ist. Zur Behandlung von seborrhoischer Haut und fetten Haaren können diese in allen üblichen Applikationsformen, wie Haarwässern, Haarshampoos, Haarkuren, Haarspülungen, Hautlotionen oder Schüttelmixturen, eingesetzt werden. Bevorzugt ist die Verwendung in Haarpflegemitteln. Neben der erfindungsgemäßen Wirkstoffkombination können diese kosmetischen Mittel übliche Träger- und Hilfsstoffe, wie Wasser, organische Lösungsmittel, oberflächenaktive Verbindungen, Öle, Fette, Wachse, Duftstoffe, Farbstoffe, Konservierungsmittel und dergleichen enthalten. Die neuen sebosuppressiven Mittel enthalten zweckmäßig 0,005—5 Gew.-%, vorzugsweise 0,01—2 Gew.-% der beanspruchten Benzoesäureesterderivate.

Herstellungsbeispiele für Verbindungen der Gruppe (I)

A) 4-(2-Ethyl-hexyloxy)-benzoesäure-methylester

Die Mischung aus 51,7 g (0,34 Mol) 4-Hydroxy-benzoesäuremethylester, 500 ml Dimethylformamid, 60 g (0,40 Mol) 2-Ethyl-hexylchlorid und 21,8 g (0,04 Mol) Natriummethyllat wurde 7 Std. zum Sieden erhitzt und nach Abkühlen und Abfiltrieren des Natriumchlorids unter vermindertem Druck zur Trockne gedampft. Der Rückstand wurde in Methylenchlorid aufgenommen und an Kieselgel (Merck) chromatographiert (Elutionsmittel: Methylenchlorid). Aus DC-reinen Fraktionen wurden 43 g (48% d. Th.) 4-(2-Ethylhexyloxy)-benzoesäure-methylester vom Brechungsindex $n_D^{20} = 1,5050$ erhalten.

In analoger Weise wurden erhalten:

B) 4-(Isononyloxy)-benzoesäure-methylester (= 4-(3,5,5-Trimethylhexyloxy)-benzosäure-methylester)
$n_D^{20} = 1,5030$, Sdp. 142—144°C/0,013 mbar

C) 4-(2-Hexyl-decyloxy)-benzoesäuremethylester,
$n_D^{20} = 1,5050$

D) 4-Isooctadecyloxy-benzoesäure-methylester (= 4-[2-(4,4-Dimethyl-2-pentyl)-5,7,7-trimethyl-1-octyloxy]-benzosäure-methylester)
$n_D^{20} = 1,4927$

E) 4-(4-tert. Butyl-benzyloxy)-benzoesäure-methylester, Schmp. 62—64°C.

I) 4-Isotridecyloxy-benzosäure-methylester
$n_D^{20} = 1,5011$, Sdp. 197—200°C/0,74 mbar

Die Herstellung erfolgt ausgehend von Isotridecanol (einem Oxoalkohol aus Tetrapropylen) über Isotridecylchlorid.

Herstellungsbeispiel für Verbindungen der Gruppe (II)

F) 4-(2-Dodecyloxy-ethoxy)-benzoesäure-methylester

Die Mischung aus 20,4 g (0,134 Mol) 4-Hydroxy-benzoesäure-methylester, 200 ml Dimethylformamid, 40 g (0,160 Mol) 2-Dodecyloxy-ethylchlorid (aus 2-Dodecyloxy-ethanol und Thionylchlorid) und 8,6 g (0,160 Mol) Natriummethylat wurde 7 Stdn. zum Sieden erhitzt und nach Abkühlen und Abfiltrieren des Natriumchlorids zur Trockne gedampft. Der Rückstand wurde in Methylenchlorid aufgenommen und säulenchromatographiert (Kieselgel, Merck/Methylenchlorid). Die nach DC vereinigten Fraktionen ergaben nach Umkristallisieren aus Methanol 30 g (61% d. Th.) reinen 4-(2-Dodecyloxy-ethoxy)-benzoesäure-methylester vom Schmp. 40—42°C.

G) 4-[2-(2-Dodecyloxy-ethoxy)-ethoxy]-benzoesäure-methylester
Wurde analog A) hergestellt.
Brechungsindex $n_D^{20} = 1,4740$

H) 4-(2-Dodecyloxy-ethoxy)-benzoesäure-3-hydroxypropylester

Die Mischung aus 12 g (0,03 Mol) 4-(2-Dodecyloxy-ethoxy)benzosäure-methylester, 60 ml Propan-1,3-diol und 0,2 g Natrimmethylat wurde 6 Stdn. auf 150°C erwärmt, wobei Methanol abdestillierte. Nach Abkühlen wurde in Methylenchlorid gelöst, mit Wasser ausgewaschen, mit Natriumsulfat getrocknet und eingedampft. Es wurden 13 g (95% d. Th.) 4-(2-Dodecyloxy-ethoxy)-benzoesäure-3-hydroxypropylester vom Schmp. 38—40°C erhalten. Nach Umkristallisieren aus Petrolether steigt der Schmp. auf 40—42°C.

Die antiseborrhoische Wirkung wurde durch nachfolgend beischriebene Trierversuche näher untersucht.

Als Versuchstiere dienten männliche Wistar-Ratten mit einem Körpergewicht von 220—230 g zu Versuchsbeginn. Beurteilt wurde visuell der Bräunungsgrad auf dem Rücken der dort geschorenen Ratten.

Die Bräunung wird durch das braune Hautoberflächenlipid der Ratten hervorgerufen. Dieser Test geht von der Beobachtung aus, daß junge weibliche Ratten sowie männliche Ratten nach dem Waschem mit Tensidlösung bzw. einem Lipidlösungsmittel und auch männliche Ratten, die systematisch mit Östrogen behandelt wurden, nur die normale helle, rosafarbene Haut nach dem Scheren aufweisen; parallel dazu sind aus den abgeschnittenen Haaren nur noch vergleichsweise sehr geringe Lipidmengen zu extrahieren.

Zur Beurteilung der Wirksamkeit wurden die Prüfsubstanzen in alkoholischer Lösung jeweils 6 Ratten halbseitig auf das Rückenfell gepinselt. Die andere Seite wurde nur mit den Lösungsmittel ohne Wirkstoffe behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Bewertungsmethoden:

Als 1. Kriterium wurde der Unterschied zwischen rechter und linker Seite gewertet, wobei pro Beurteiler und Tier jeweils 1 Punkt nu vergeben war, und zwar in der Weise, daß die
dunklere Seite mit 1 Punkt
hellere Seite mit 0 Punkte und
bei Gleichheit beide Seiten mit 0,5 Punkte benotet wurden.

Signifikante Differenzen zwischen unbehandelter und behandelter Seite nach dieser Bewertungsmethode zeigen die lokale Wirksamkeit einer Substanz an.

Als 2. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach dieser Bewertungsmethode werden die Punktsummendifferenzen zwischen den unbehandelten Kontrolltieren und jeweils den behandelten und unbehandelten Seiten ($\Delta P$) der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinflüß.

Zur Differenzierung der Effekte gemäß Beurteilungsmethode 1 und 2 wurde das folgende Schema verwendet:

| Zeichen | Punktdifferenz |
|---|---|
| ++ | sehr groß (≥ 99,9% Wahrscheinlichkeit) |
| + | signifikant (≥ 95% Wahrscheinlichkeit) |
| – | (< 95% Wahrscheinlichkeit) |

Prozentuale Sebumreduktion

Die Sebumreduktion erreichnet sich aus der Punktedifferenz in der Weise, daß man den Quotienten aus der Punktedifferenz $\Delta P$ und der Punktezahl für die Kontrolgruppe $P_k$ bildet und den erhaltenen Wert in % angibt.

$$\text{Sebumreduktion} = \frac{\Delta P}{P_k} \cdot 100\ [\%]$$

Die Benzoesäureesterderivate wurden in Konzentrationen von 0,01 bis 0,5% in Alkohol in der angegebenen Weise appliziert. Die ergebnisse sind in der Tabelle zusammengefaßt.

TABELLE
Bewertung der sebosuppressiven Effekte

| Substanz | % Sebumreduktion bei Anwendungskonzentration | | |
|---|---|---|---|
| | 0,1% | 0,05% | 0,02% |
| B | 76 | 36 | — |
| E | — | 77 | 10 |
| F | — | 63 | — |
| H | — | 71 | — |
| I | — | 83 | 83 |
| 4-Dodecyloxy-benzoesäuremethylester DE—OS 31 21 064 | 18 | 0 | — |
| 4-Dodecyloxy-benzoesäure-3-hydroxypropylester DE—OS 33 01 313 | 33 | 18 | — |

Aus den Werten der Tabelle wird die überlegene Wirkung der beanspruchten Substanzen deutlich.

Beispiele für Rezepturen

Nachfolgend werden für die erfindungsgemäßen topischen Mittel zur Behandlung von stark fettendem Haar und seborrhoischer Haut Rezepturen gegeben (in Gewichtsprozent)

Haarwasser

| | |
|---|---|
| Ethanol (96% ig) oder Isopropanol | 54,0% |
| Substanz B oder H | 0,2% |
| Parfümöl | 0,5% |
| Wasser | 45,3% |

Shampoo für fettendes Haar

| | |
|---|---|
| Ammoniumlaurylsulfat mit 33—35% Aktivsubstanz | 42,0 |
| Kokosfettsäurediethanolamid | 3,0 |
| Natriumchlorid | 2,0 |
| Natriumsulfat | 2,0 |
| Substanz E oder F | 1,5 |
| Konservierungsmittel | 0,1 |
| Parfümöl | 0,1 |
| Wasser | 49,3 |

Haarkur

| | |
|---|---|
| Glycerinmono-distearat | 0,7 |
| kationisches Tensid | 2,0 |
| Cholesterin | 0,2 |
| Sojalecithin | 0,3 |
| Emulgade A(R) (Gemisch von Cetylstearylalkohol mit nichtionogenen Emulgatoren) | 8,0 |
| Parfümöl | 0,3 |
| Substanz B oder F | 3,0 |
| Wasser, vollentsalzt | 85,5 |

Hautcreme

| | |
|---|---|
| Selbstemulgierendes Gemisch aus Mono/ Diglyceriden höherer gesättigter Fettsäuren mit Kaliumstearate (Cutina KD 16(R), Henkel KGaA) | 16,0 |
| Cetylstearylalkohol mit ca. 12 Mol Ethylenoxid | 1,0 |
| 2-Octyldodecanol | 6,0 |
| Isopropylmyristat | 4,0 |
| Glycerin | 6,0 |
| Substanz E oder G | 4,0 |
| Wasser | 63,0 |

**Patentansprüche**

1. Sebosuppressive kosmetische Mittel, gekennzeichnet durch einen Gehalt an Benzosäureesterderivaten der allgemeinen Formel

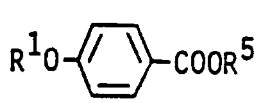

worin bedeuten
(I) $R^1$ = verzweigtkettiges $C_6$—$C_{18}$-Alkyl, $C_3$—$C_9$-Alkylbenzyl, oder
(II) $R^1$ = $R^2$—(O—Y)$_n$—
$R^2$ = $C_6$—$C_{18}$-Alkyl, $C_3$—$C_9$-Alkylbenzyl
Y = $CHR^3$—$CHR^4$,
$R^3$, $R^4$ = H, Methyl
n = 1 oder 2
$R^5$ = $C_1$—$C_4$-Alkyl, Methoxy-, oder Ethoxy alkyl, $C_1$—$C_4$-Hydroxyalkyl.

2. Mittel nach Anspruch 1, gekennzeichnet durch einen Gehalt an Benzoesäureesterderivaten der angegebenen allgemeinen Formel, worin bedeuten
(I) $R^1$ = verzweigtkettiges $C_8$—$C_{13}$-Alkyl, oder $C_3$—$C_6$-Alkylbenzyl oder insbesondere mehrfach verzweigtes $C_8$—$C_{13}$-Alkyl
oder

(II) $R^1 = R^2$—$(O$—$Y)_n$
$R^2 = C_9$—$C_{14}$-Alkyl
$n = 1$
Y wie in Anspruch 1
$R^5$ wie in Anspruch 1

3. Mittel nach Anspruch 1 oder 2, gekennzeichnet durch einen Gehalt an
4-(Isononyloxy)-benzoesäure-methylester, oder
4-(4-tert. Butyl-benzyloxy)-benzoesäure-methylester oder
4-(2-Dodecyloxy-ethoxy)-benzosäure-methylester oder
4-(2-Dodecyloxy-ethoxy)-benzoesäure-3-hydroxypropylester.

4. Mittel nach Anspruch 1 bis 3, gekennzeichnet durch einen Gehalt von 0,005 bis 5 Gewichtsprozent, vorzugsweise 0,01 bis 2 Gewichtsprozent an Benzoesäureesterderivaten.

5. Verbindungen der allgemeinen Formel

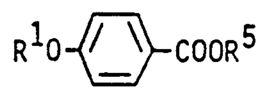

worin bedeuten
(I) $R^1$ = verzweigtkettiges $C_6$—$C_{18}$-Alkyl, $C_3$—$C_9$-Alkylbenzyl oder
(II) $R^1 = R^2$—$(O$—$Y)_n$—
$R^2 = C_6$—$C_{18}$-Alkyl, $C_3$—$C_9$-Alkylbenzyl
$Y = CHR^3$—$CHR^4$
$R^3$, $R^4$ = H, Methyl
n = 1 oder 2
$R^5 = C_1$—$C_4$-Alkyl, Methoxy- oder Ethoxy-alkyl, $C_1$—$C_4$-Hydroxyalkyl.

6. Verbindungen der allgemeinen Formel

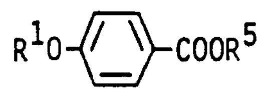

worin bedeuten
(I) $R^1$ = verzweigtkettiges $C_8$—$C_{13}$-Alkyl oder $C_3$—$C_6$-Alkylbenzyl oder insbesondere mehrfach verzweigtes $C_8$—$C_{13}$-Alkyl
oder
(II) $R^1 = R^2$—$(O$—$Y)_n$
$R^2 = C_9$—$C_{14}$-Alkyl
$n = 1$
Y wie in Anspruch 1
$R^5$ wie in Anspruch 1

7. 4-(Isononyloxy)-benzoesäure-methylester, oder
4-(4-tert. Butyl-benzyloxy)-benzoesäure-methylester oder
4-(2-Dodecyloxy-ethoxy)-benzoesäure-methylester oder
4-(2-Dodecyloxy-ethoxy)-benzoesäure-3-hydroxypropylester.

**Revendications**

1. Compositions cosmétiques sébosuppressives caractérisées par une teneur en dérivés d'esters d'acide benzoïque de formule générale

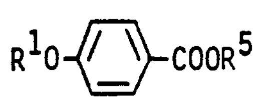

(I) $R^1$ représente un groupe alkyle en $C_6$—$C_{18}$ à chaîne ramifiée ou un groupe alkyl($C_3$—$C_9$)— benzyle,
ou
(II) $R^1 = R^2$—$(O$—$Y)_n$—
$R^2$ étant un groupe alkyle en $C_6$—$C_{18}$ ou alkyl ($C_3$—$C_9$)-benzyle,
$Y = CHR^3$—$CHR^4$,
$R^3$, $R^4$ = H, méthyle,
n = 1 ou 2

$R^5$ représente un groupe alkyle en $C_1$—$C_4$, méthoxy-, éthoxyalkyle, hydroxyalkyle en $C_1$—$C_4$.

2. Produits selon la revendication 1, caractérisé par une teneur en dérivés d'esters d'acide benzoïque de formule générale indiquée, dans laquelle

(I) $R^1$ représente un groupe alkyle en $C_8$—$C_{13}$ à chaîne ramifiée ou alkyl ($C_3$—$C_6$)-benzyle ou en particulier un groupe alkyle en $C_8$—$C_{13}$ plusieurs fois ramifié, ou

(II) $R^1 = R^2$—(O—Y)$_n$

$R^2$ étant un groupe alkyle en $C_9$—$C_{14}$,

n = 1

Y est tel que dans la revendication 1

$R^5$ est tel que dans la revendication 1.

3. Produits selon la revendication 1 ou 2, caractérisés par une teneur en

4-(isononyloxy)-benzoate de méthyle ou

4-(4-tert-butyl-benzyloxy)-benzoate de méthyle ou

4-(2-dodécyloxy-éthoxy)-benzoate de méthyle ou

4-(2-dodécyloxy-éthoxy)-benzoate de 3-hydroxypropyl.

4. Produits selon l'une quelconque des revendications 1 à 3, caractérisés par une teneur de 0,005 à 5% en poids, de préférence de 0,01 à 2% en poids, en dérivés d'esters d'acide benzoïque.

5. Composés de form générale

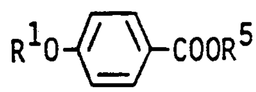

dans laquelle

(II) $R^1$ représente un groupe alkyle en $C_6$—$C_{18}$ à chaîne ramifiee ou un groupe alkyl($C_3$—$C_9$)-benzyle, ou

(II) $R^1 = R^2$—(O—Y)$_n$—

$R^2$ étant un groupe alkyle en $C_6$—$C_{18}$ ou alkyl ($C_3$—$C_9$)-benzyle,

Y = $CHR^3$—$CHR^4$,

$R^3$, $R^4$ = H, méthyle,

n = 1 ou 2.

$R^5$ représente un groupe alkyle en $C_1$—$C_4$, méthoxy-, éthoxyalkyle, hydroxyalkyle en $C_1$—$C_4$.

6. Composés de formule générale

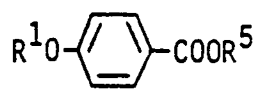

dans laquelle

(I) $R^1$ représente un groupe alkyle en $C_8$—$C_{13}$ à chaîne ramifiée ou alkyl ($C_3$—$C_6$)-benzyle ou en particulier un groupe alkyle en $C_8$—$C_{13}$ plusieurs fois ramifié, ou

(II) $R^1 = R^2$—(O—Y)$_n$

$R^2$ étant un groupe alkyle en $C_9$—$C_{14}$,

n = 1

Y est tel que dans la revendication 1

$R^5$ est tel que dans la revendication 1.

7. 4-(isononyloxy)-benzoate de méthyle ou

4-(4-tert-butyl-benzyloxy)-benzoate de méthyle ou

4-(2-dodécyloxy-éthoxy)-benzoate de méthyle ou

4-(2-dodécyloxy-éthoxy)-benzoate de 3-hydroxypropyle.

**Claims**

1. Sebo-suppressive cosmetic preparations which are characterized by a content of benzoic acid ester derivatives corresponding to the following general formula

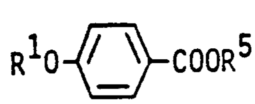

in which

(I) $R^1$ = branched $C_{6-18}$ alkyl, $C_{3-9}$ alkylbenzyl or
(II) $R^1$ = $R^2$—(O—Y)$_n$—
$R^2$ = $C_{6-18}$ alkyl, $C_{3-9}$ alkylbenzyl,
Y = $CHR^3$—$CHR^4$,
$R^3$, $R^4$ = H, methyl
n = 1 or 2
$R^5$ = $C_{1-4}$ alkyl, methoxy or ethoxyalkyl, $C_{1-4}$ hydroxyalkyl.

2. Preparations as claimed in claim 1, characterized by a content of benzoic acid ester derivatives corresponding to the above general formula in which
(I) $R^1$ = branched chain $C_{8-13}$ alkyl or $C_{3-6}$ alkylbenzyl or, in particular repeatedly branched $C_{8-13}$ alkyl or
(II) $R^1$ = $R^2$—(O—Y)$_n$
$R^2$ = $C_{9-14}$ alkyl,
n = 1,
Y is as defined in claim 1,
$R^5$ is as defined in claim 1.

3. Preparations as claimed in claim 1 or 2, characterized by a content of
4-(isononyloxy)-benzoic acid methyl ester or
4-(4-tert. butylbenzyloxy)-benzoic acid methyl ester or
4-(2-dodecyloxy-ethoxy)-benzoic acid methyl ester or
4-(2-dodecyloxy-ethoxy)-benzoic acid-3-hydroxypropyl ester.

4. Preparations as claimed in claims 1 to 3, characterized by a content of from 0.005 to 5% by weight and preferably of from 0.01 to 2% by weight of benzoic acid ester derivatives.

5. Compounds corresponding to the following general formula

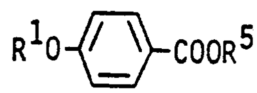

in which
(II) $R^1$ = branched $C_{6-18}$ alkyl, $C_{3-9}$ alkylbenzyl or
(II) $R^1$ = $R^2$—(O—Y)$_n$—
$R^2$ = $C_{6-18}$ alkyl, $C_{3-9}$ alkylbenzyl,
Y = $CHR^3$—$CHR^4$,
$R^3$, $R^4$ = H, methyl
n = 1 or 2
$R^5$ = $C_{1-4}$ alkyl, methoxy or ethoxyalkyl, $c_{1-4}$ hydroxyalkyl.

6. Compounds corresponding to the following general formula

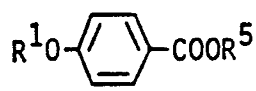

(I) $R^1$ = branched chain $C_{8-13}$ alkyl or $C_{3-6}$ alkylbenzyl or, in particular repeatedly branched $C_{8-13}$ alkyl or
(II) $R^1$ = $R^2$—(O—Y)$_n$
$R^2$ = $C_{9-14}$ alkyl,
n = 1,
Y is as defined in claim 1,
$R^5$ is as defined in claim 1.

7. 4-(isononyloxy)-benzoic acid methyl ester or
4-(4-tert. butylbenzyloxy)-benzoic acid methyl ester or
4-(2-dodecyloxy-ethoxy)-benzoic acid methyl ester or
4-(2-dodecyloxy-ethoxy)-benzoic acid-3-hydroxypropyl ester.